# EUROPÄISCHE PATENTSCHRIFT

(11) **EP 1 859 752 B1**
(45) Veröffentlichungstag und Bekanntmachung des Hinweises auf die Patenterteilung: **13.08.2008**
(21) Anmeldenummer: 06010835.4
(22) Anmeldetag: 26.05.2006
(51) Int. Cl.: A61B 17/80

(54) **Knochenplatte**
Bone plate
Plaque d'ostéosynthèse

(43) Veröffentlichungstag der Anmeldung: 28.11.2007
(73) Patentinhaber: AAP Implantate AG, 12099 Berlin (DE)
(72) Erfinder: Ahrens, Uwe, 10179 Berlin (DE); Bakhshi, Majid, 12053 Berlin (DE); Dr. Fischer, Hans-Joachim, 12277 Berlin (DE)
(74) Vertreter: von Eichel-Streiber, Caspar

(56) Entgegenhaltungen:
- WO-A-01/54601

## Beschreibung

Die Erfindung betrifft eine Knochenplatte.

Knochenplatten sind beispielsweise bekannt aus der CH 462375, der WO 2000/53111, der WO 2001/54601 oder der EP 0760632 B1.

Die Ausgestaltung der Löcher ist bei diesem Stand der Technik so, dass sie aus der Kombination von zwei sich überschneidenden Löchern bestehen mit unterschiedlichen Durchmessern und zu einem Durchgangsloch kombiniert sind und jede Seite des Durchgangslochs mit seinem festen Durchmesser seine eigene Funktionalität aufweist und auch getrennt voneinander betrachtet werden muss.

So ist es beispielsweise nicht möglich, auf der gewindeanteiligen Seite des Durchgangsloches eine Standard-Kortikalisschraube einzubringen, sondern nur eine Schraube mit Kopfgewinde.

Die andere Seite des Durchgangsloches, die keinen Gewindeanteil aufweist, ist nur für die Einbringung einer Standard-Kortikalisschraube geeignet, die auch mit Kompression und Winkelvariabilität appliziert werden kann.

Somit könnte man die Löcher auch völlig voneinander trennen da sie keine funktionellen Schnittpunkte aufweisen.

Dieser Nachteil zieht sich dann auch durch die gesamte Anwendung hin, denn beim Einbringen einer Standard-Kortikalisschraube muss auch darauf geachtet werden, dass man sich auch immer auf der richtigen Seite des Durchgangsloches befindet.

Ausgehend hiervon ist es die Aufgabe der Erfindung, die Einsatzmöglichkeiten einer Knochenplatte zu verbessern und deren Flexibilität in der Handhabung zu erhöhen.

Gelöst wird diese Aufgabe durch eine Knochenplatte mit den Merkmalen des Anspruchs 1. Vorteilhafte Weiterbildungen sind in den abhängigen Ansprüchen 2 bis 9 angegeben. In Anspruch 10 schließlich ist eine zu der erfindungsgemäßen Knochenplatte passende Knochenschraube angegeben, die mit der Platte zusammen als ein Fixierungssystem angesehen werden kann.

Erfindungswesentlich ist für die neuartige Knochenplatte, dass in einer Richtung quer zu der Ebene ihrer Oberseite gesehen gleichermaßen übereinander angeordnet in einem Teilabschnitt sowohl ein Gewinde als auch eine glattwandige Haltestruktur angeordnet sind. Durch das Zusammenwirken von Gewinde und glattwandiger Haltestruktur kann eine mit entsprechenden Gegenstrukturen ausgebildete Knochenschraube besonders effektiv lagefixiert in der Knochenplatte gehalten und gegen Verkippungen und Verkantungen gesichert werden. Durch das Zusammenwirken des Gewindes in dem Langloch mit einem entsprechend ausgebildeten Gegengewinde am Kopf bzw. Hals der Knochenschraube wird die Knochenschraube mit einer korrespondierend zu der Haltestruktur ausgebildeten Gegenstruktur bzw. Anlagestruktur fest in diese Haltestruktur gepresst und so sicher fixiert.

Ferner erfindungswesentlich ist die Tatsache, dass bei der erfindungsgemäßen Knochenplatte sich die Gewindezüge entlang eines kürzeren Abschnittes des Langloches erstrecken als die glattwandige Haltestruktur. Diese technische Maßnahme erlaubt im Gebrauch ein einfaches und sicheres Eingleiten einer an ihrem Hals und/oder Kopf mit einem Gewinde sowie mit der Gegenstruktur versehenen Knochenschraube in die an dem Langloch angeordneten Strukturen (Gewinde, Haltestruktur) bei einem kontinuierlichen Verschieben der Knochenschraube entlang dem Langloch. Ein gleitender Übergang in eine glattwandige Haltestruktur ist einfacher zu realisieren als ein seitliches Hineingleiten in ein Gewinde. So kann die Führung für einen sicheren und passgenauen Übergang des Gewindes an dem Kopf und/oder Hals der Knochenschraube durch das zuerst einsetzende, richtungsleitende Zusammenwirken zwischen Haltestruktur und Gegenstruktur bewerkstelligt werden.

Bevorzugte Winkelbereich für die Umschlingung des am Kopf und/oder Hals der Knochenschraube angeordneten, in Eingriff mit dem Gewinde des Langloches stehenden Gewindes sind in Anspruch 2 angegeben, solche für die entsprechende Umschlingung der Gegenstruktur in Anspruch 3. Diese Werte haben sich in Versuchen als am besten geeignet für ein vorzugsweise durchgängiges Verschieben der Schraube entlang des Langloches bei einem dennoch gewährleisteten sicheren Halt einer in dem Gewinde/der Haltestruktur des Langloches festgelegten Knochenschraube. Eine freie Verschiebbarkeit einer Knochenschraube entlang der Längsachse des Langloches ermöglicht, dass diese sowohl zum Aufbringen einer auf die Frakturstelle hingerichteten Anpresskraft als auch zum winkelfesten Fixieren der Platte verwendet werden kann.

Derzeit wird als Haltestruktur eine konische Fläche bevorzugt (Anspruch 4). In dieser kann eine entsprechend konisch ausgebildete Oberfläche (Gegenstruktur) des Kopfes bzw. Halses der Knochenschraube festgelegt werden. Prinzipiell ist es auch möglich, andere Formen der Oberflächen der Haltestrukturen einzusetzen, beispielsweise teilsphärische Flächen.

Bei der erfindungsgemäßen Knochenplatte ist es grundsätzlich egal, ob von der Oberseite der Platte her gesehen die Haltestruktur zuerst und im unteren Bereich der Knochenplatte das Gewinde angeordnet ist oder umgekehrt. Ebenso gut können wechselnd und jeweils unterbrochen durch wenigstens einen Abschnitt mit einer Haltestruktur mehrere Gewindeabschnitte in der Knochenplatte vorgesehen sein. Bevorzugt wird ein Ausführungsbeispiel, bei dem der Gewindeabschnitt nahe der Oberseite der Knochenplatte liegt und eine Haltestruktur nahe der Unterseite der Knochenplatte (vgl. Anspruch 5).

In einer Ausgestaltung gemäß Anspruch 6 ist vorgesehen, dass das Langloch an der Oberseite der Knochenplatte eine erste Führungsstruktur aufweist. Diese dient der Führung des Kopfes oder Halses einer an ihrem Hals und/oder Kopf mit dem Gewinde und der Gegenstruktur versehenen Knochenschraube. Diese erste Führungsstruktur kann beispielsweise ein umlaufender Rand mit teilkreisförmig ausgebildetem Querschnitt sein, in dem eine entsprechend sphärisch bzw. teilsphärisch ausgebildete Struktur am Kopf bzw. Hals einer Knochenschraube gleiten kann, z.B. die äußere Konturlinie des an dem Kopf und /oder Hals der Knochenschraube ausgebildeten Gewindes. Gemäß Anspruch 6 definiert die erste Führungsstruktur eine Führungsbahn. Diese Führungsbahn ist eine solche Bahn, entlang derer ein gedachter Punkt am Schraubenkopf gleitet, wenn die Schraube entlang der Führungsstruktur verschoben wird. Ferner wird diese Führungsbahn von einer Längsachse des Gewindes, dies ist im Sinne der Erfindung die Achse, um die ein in das Gewinde einzuschraubendes Gegengewinde bei einem Einschraubvorgang rotiert, um einen von 90° verschiebenden Winkel geschnitten. Diese Art der Verkippung zwischen Gewinde und Führungsbahn bewirkt, dass auch bei einer in der Ebene der Oberfläche gelegenen Umschlingung von maximal 180° das Gewinde respektive die Haltestruktur dem Schraubkopf bzw. -hals einen sicheren Halt verleiht, da die Verkippung zu einer effektiv weitergehenden Umschlingung zumindest in einem Teilbereich des Gewindes führt. Dieser Effekt wird durch eine aufgrund der Verkippung ausgelöste "Klemm-" bzw. "Verkeilwirkung" erzielt. Unterstützt wird der die lagegetreue Fixierung der Knochenschraube dabei noch durch das Zusammenwirken zwischen der erfindungsgemäßen Haltestruktur und der Gegenstruktur an dem Hals und/oder Kopf der Knochenschraube.

Ferner wird bevorzugt, dass die Führungsbahn um einen Winkel zwischen 0° und 90° gegenüber der Plattenebene geneigt ist (Anspruch 7). Hier wird ferner eine Ausführungsvariante bevorzugt, bei welcher das Gewinde bzw. die Haltestruktur in einem Abschnitt des Langloches geordnet sind, in dem die Führungsbahn am tiefsten, d.h. am weitesten von der Oberseite der Knochenplatte entfernt, liegt (vgl. Anspruch 8).

Der mit Gewinde und Haltestruktur versehene Abschnitt des Langloches ist erfindungsgemäß auf einen Teilabschnitt des Langloches beschränkt, er liegt vorzugsweise an einer Schmalseite des Langloches (vgl. Anspruch 8).

Mit Vorteil weist das Langloch der erfindungsgemäßen Knochenplatte neben der oben beschriebenen ersten Führungsstruktur noch eine zweite Führungsstruktur, die der Führung von Knochenschrauben mit gewindelosem Kopf und Hals (Standard-Kortikalisschrauben) dient. Diese Trennung der Führungsstrukturen erlaubt eine saubere Trennung in der Wirkung der beiden möglichen mit der erfindungsgemäßen Knochenplatte zu verwendenden Knochenschrauben.

Unter Langloch wird im Sinne dieser Erfindung nicht lediglich ein durch Verschieben eines Kreises entlang einer geraden Achse hervorgerufenes Lochbild verstanden, sondern grundsätzlich jegliche Öffnungsform, die in einer ersten Erstreckungsrichtung länger ist als in einer zweiten Erstreckungsrichtung. Somit kann ein Langloch im Sinne der Erfindung auch "sich dreieckig verjüngend" ausgebildet sein, oval oder "schlüssellochförmig". Vorzugsweise ist dieses Langloch durchgehend, d.h. ohne in das Loch hineinragende "Barrieren", ausgebildet.

Die Knochenplatte kann mit einer speziell mit einem Gewinde und einer Gegenstruktur an dem Schraubenkopf und/oder -hals ausgebildeten Knochenschraube zugleich winkelstabil fixiert und gespannt werden. Es soll hier jedoch betont werden, dass die erfindungsgemäße Knochenplatte auch mit Standardknochenschrauben bzw. an solche Standardknochenschrauben angelehnten Knochenschrauben fixiert werden kann, die insbesondere weder ein Gewinde noch eine Gegenstruktur an ihrem Schraubenkopf bzw. -hals aufweisen.

Die erfindungsgemäße Knochenplatte bietet - auch zusammen mit der erfindungsgemäßen Knochenschraube - eine große Vielzahl von Einsatzmöglichkeiten, die sie zum Fixieren von unterschiedlichsten Frakturen geeignet macht.

Hierfür wird insbesondere bevorzugt, dass sämtliche Öffnungen in der Knochenplatte als Langlöcher mit den erfindungsgemäßen Eigenschaften ausgebildet sind.

Weitere Vorteile und Merkmale der Erfindung ergeben sich aus der nachfolgenden Beschreibung eines Ausführungsbeispieles anhand der beigefügten Figuren. Dabei zeigen:

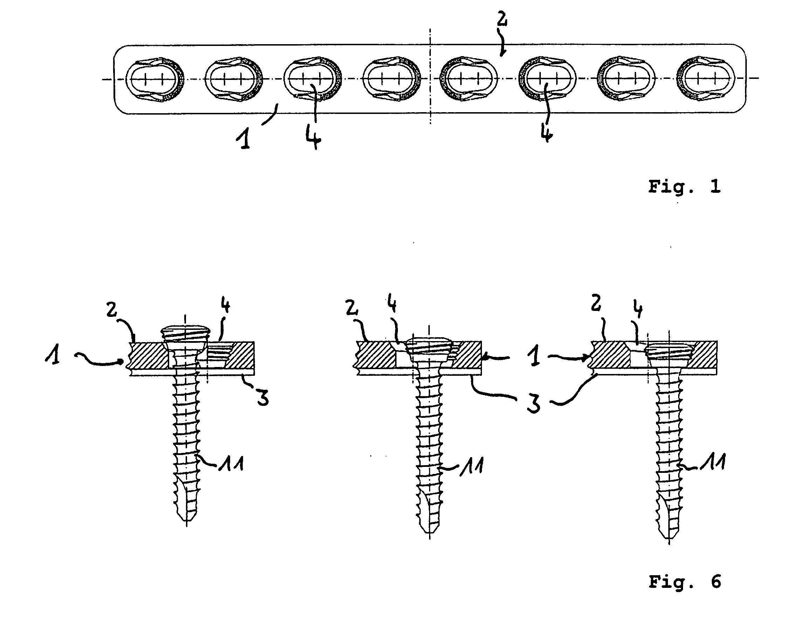

- Fig. 1: ein Ausführungsbeispiel für eine erfindungsgemäße Knochenplatte in einer Draufsicht,
- Fig. 2: in einem vergrößerten Ausschnitt die Aufsicht auf ein Langloch eines Ausführungsbeispiels einer Knochenplatte gemäß der Erfindung,
- Fig. 3: in einer vergrößerten Darstellung ein Längsschnitt durch das Langloch gemäß Fig. 2,
- Fig. 4: a) eine Ansicht einer Knochenschraube mit an ihrem Schraubenkopf ausgebildetem Gewinde und Gegenstruktur zum Zusammenwirken mit dem Gewinde und der Haltestruktur in der erfindungsgemäßen Knochenplatte und b) eine Ansicht einer Standardknochenschraube,
- Fig. 5: a) eine mit Fig. 4 a) vergleichbare Ansicht einer Knochenschraube zum Zusammenwirken mit dem Gewinde und der Haltestruktur in der erfindungsgemäßen Knochenplatte und b) eine Ansicht einer Standardknochenschraube in einer möglichen Stellung in dem Langloch zur Fixierung der Knochenplatte,
- Fig. 6: in einer aus drei Einzeldarstellungen bestehenden Sequenz eine Veranschaulichung des Eingleitens der mit Gewinde und Gegenstruktur an ihrem Schraubenkopf versehenen Knochenschraube in den Abschnitt des Langloches mit Gewinde und Haltestruktur,

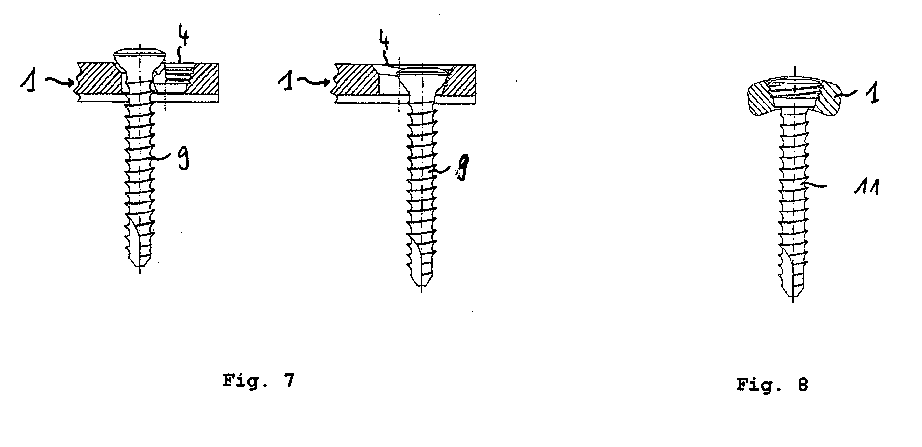

- Fig. 7: in einer ähnlichen Darstellung wie Fig. 6 in zwei Einzeldarstellungen eine Sequenz zur Veranschaulichung des Eingleitens einer Standardknochenschraube in den Abschnitt des Langloches mit Gewinde und Haltestruktur,
- Fig. 8: den festen Sitz einer mit Gewinde und Gegenstruktur versehenen Knochenschraube in dem Gewinde und der Haltestruktur des Langloches in einem Schnitt quer zu der Längserstreckung der Knochenplatte,
- Fig. 9: a) bis c) drei unterschiedliche, mögliche weitere Formen eines Langloches einer erfindungsgemäßen Knochenplatte.

In den Figuren sind schematisch Ausführungsbeispiele bzw. -formen einer erfindungsgemäßen Knochenplatte gezeigt. Dabei sind in den Figuren gleiche bzw. funktionsgleiche Elemente mit gleichen Bezugszeichen versehen. Die gezeigten Ausführungsbeispiele sind keinesfalls maßstabsgerecht, sondern dienen lediglich einer prinzipiellen Erläuterung.

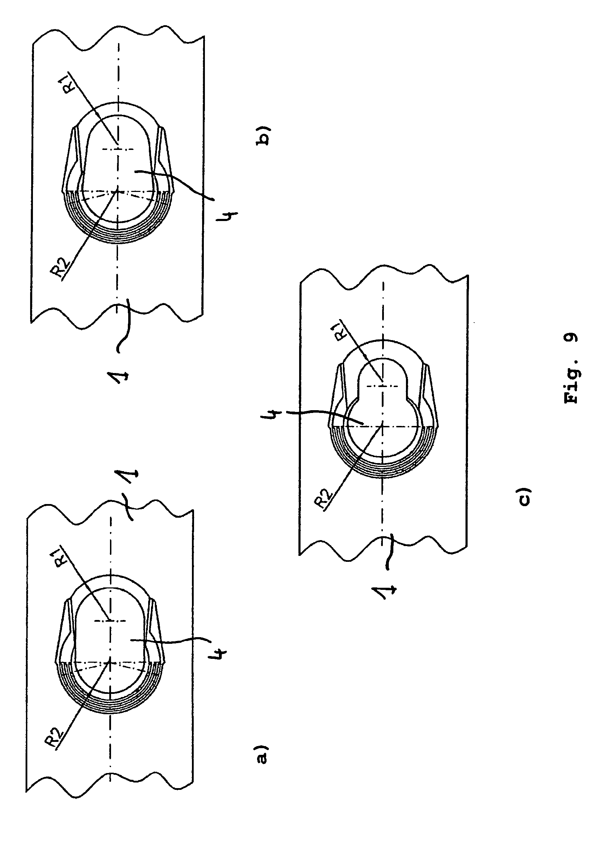

Eine erfindungsgemäße Knochenplatte 1 hat eine Oberseite 2 und eine Unterseite 3, welche zur Auflage auf den zu fixierenden Knochen dient. Zwischen Oberseite 2 und Unterseite 3 sind durchgehende Öffnungen in Form von Langlöchern 4 in der Knochenplatte 1 eingebracht. Die erfindungsgemäße Knochenplatte 1 weist in diesem Ausführungsbeispiel insgesamt acht Langlöcher 4 auf, ohne jedoch auf diese Anzahl beschränkt zu sein. Die Langlöcher 4 sind in dem bevorzugten Ausführungsbeispiel sich zu einer der Längsseiten hin verjüngend ausgebildet. Sie können jedoch, wie beispielsweise in den alternativen Ausgestaltungen gemäß Fig. 9a) bis 9c) gezeigt, auch gerade verlaufen und der Form entsprechen, wie sie durch Verschieben eines Kreises entlang einer vorgegebenen Strecke entsteht, zur Mitte der Knochenplatte hin verjüngt verlaufen oder die Form eines "Schlüsselloches" annehmen (Fig. 9c)). Selbstverständlich sind auch alle anderen denkbaren Formen von Langlöchern möglich. Bevorzugt werden solche Formen, die durchgehend, d.h. in ihrem Inneren hindernisfrei gestaltet sind.

An einer Stirnseite eines jeden Langloches sind Gewindezüge 5 sowie eine Haltestruktur 6 gebildet. Die Gewindezüge 5 und die Haltestruktur 6 liegen in einer Richtung quer zu der Plattenebene übereinander geordnet, wobei in diesem Ausführungsbeispiel die Gewindezüge 5 oben (der Oberseite 2 der Knochenplatte 1 zugewandt) und die Haltestruktur 6 unten (der Unterseite 3 der Knochenplatte 1 zugewandt) angeordnet sind. Die Haltestruktur 6 ist durch einen glattwandigen konisch sich in Richtung der Unterseite 3 der Knochenplatte 1 verjüngenden Wandabschnitt des Langloches 4 gebildet.

Wie in den Figuren zu erkennen ist, sind bei dem gezeigten Ausführungsbeispiel die Gewindezüge 5 bzw. Haltestrukturen 6 der Langlöcher 4 an Schmalseiten der letztgenannten angeordnet.

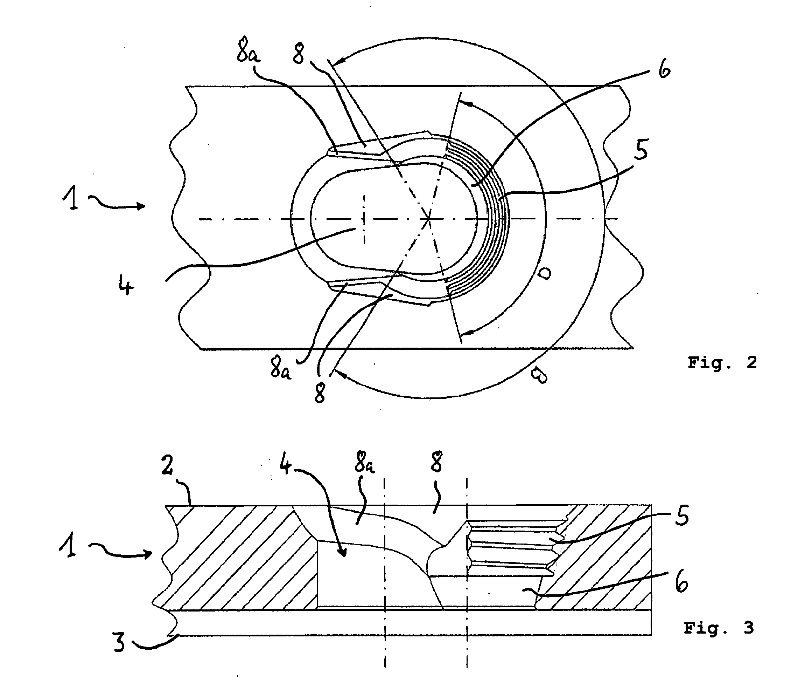

Insbesondere in Fig. 2 ist zu erkennen, dass die Gewindezüge 5 in einem kürzeren Umfangsabschnitt des Langloches 4 verlaufen als die Haltestruktur 6. Während sich erstere über einen Winkel α von vorzugsweise zwischen 60° und 190° erstrecken, verläuft die letztere über einen Winkelbereich β von bevorzugt zwischen 185° und 300°. Diese Wahl der unterschiedlichen Umschlingungsbereiche ermöglicht ein glattes Eingleiten der Knochenschraube in diesen Bereich bei einer zuverlässigen letztendlichen Fixierung, wie später noch beschrieben werden wird.

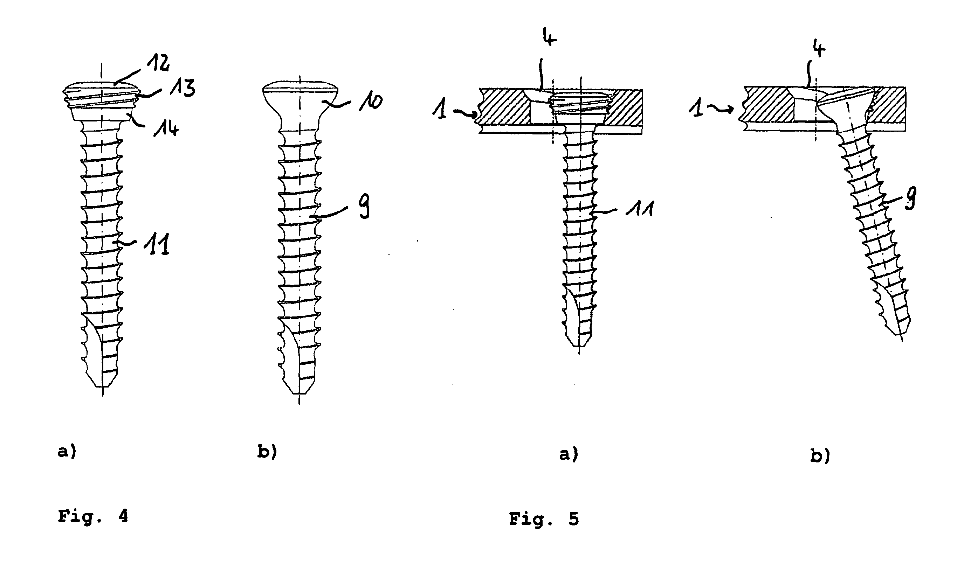

In den Figuren 4a) und b) sind eine speziell für das Zusammenwirken mit der erfindungsgemäßen Knochenplatte 1 konzipierte Knochenschraube 11 mit einem Schraubenkopf 12 bzw. eine Standard-Knochenschraube 9 mit einem glatt und an der Unterseite sphärisch geformten Schraubenkopf 10 gezeigt. Der Schraubenkopf 12 enthält an einem oberen Abschnitt ein in diesem Ausführungsbeispiel sphärisch gestaltetes Gewinde 13 und in Richtung der Längsachse der Schraube gesehen darunter einen Abschnitt mit einer Anlage- bzw. Gegenstruktur 14. Diese Anlagestruktur 14 ist in diesem Ausführungsbeispiel glattwandig gebildet mit konischem, sich in Richtung der Schraubenspitze verjüngenden Verlauf, und sie ist komplementär zu der Form der Haltestruktur 6 der Knochenplatte 1.

Das Langloch 4 enthält an seiner der Oberseite 2 der Knochenplatte 1 zugewandten Öffnung zwei Führungsstrukturen 8 und 8a. Während die erste Führungsstruktur 8 eine Führungsstruktur für die Führung einer Knochenschraube 11 mit Gewinde 13 und Anlagestruktur 14 am Schraubenkopf 12 ist, dient die Führungsstruktur 8a der Führung des Schraubenkopfes 10 einer Standardknochenschraube 9. Beide Führungsstrukturen 8, 8a sind übereinander angeordnet durch einen Rand mit teilkreisförmigem Querschnitt gebildet, der in das Langloch 4 jeweils beispielsweise durch einen spanenden Bearbeitungsschritt (z.B. Fräsen) eingebracht wird. Beide Führungsstrukturen 8, 8a definieren jeweils eine Führungsbahn, die zu der Oberfläche der Knochenplatte 1 geneigt verläuft. Insbesondere schneidet die erste Führungsstruktur 8 (bzw. die durch diese definiert Führungsbahn) die durch den Verlauf der Oberseite 2 der Knochenplatte 1 festgelegten Plattenebene unter einem von 0° und 90° verschiedenen ersten Winkel. Gleichermaßen ist die Führungsbahn gegenüber einer Gewindelängsachse ebenfalls geneigt, und zwar ebenfalls um einen von 0° und 90° verschiedenen Winkel.

In den Figuren 5b) bis 8 ist das Zusammenwirken zwischen einer Knochenschraube 9 der herkömmlichen Art bzw. einer Knochenschraube 11 der erfindungsgemäß angepassten Art und der erfindungsgemäßen Knochenplatte 1 gezeigt. So soll anhand dieser Figuren auch die Funktion der erfindungsgemäßen Knochenplatte 1 und ihrer Elemente nun erläutert werden.

In den Figuren 5b) und 7 ist in zwei unterschiedlichen Positionen eine in das Langloch 4 der Knochenplatte 1 eingesetzte, herkömmliche Knochenschraube 9 dargestellt. Diese Knochenschraube 9 liegt mit der sphärisch glatt geformten Unterseite des Schraubenkopfes 10 auf der komplementär geformten Führungsstruktur 8a des Langloches 4 auf und kann so der durch diese Führungsstruktur vorgegebenen Führungsbahn folgend entlang der Längsachse des Langloches 4 verschoben werden. Durch die Schiefstellung der Führungsbahn wird beim Einschrauben der Knochenschraube 9 eine Kompressionswirkung erzielt, indem die Knochenplatte 1 in eine Richtung gedrückt bzw. ein damit zu fixierender Knochen in die andere Richtung gezogen wird. Insbesondere ist die Au-ßenkontur des in dem Langloch 4 durch die Gewindezüge 5 gebildeten Gewindeabschnittes so geformt, dass sie ebenfalls eine komplementär zu der konisch geformten Unterseite des Schraubenkopfes 10 geformte Führungsfläche bildet. Diese Außenkontur bildet somit Bestandteil der Führungsstruktur 8a. An dieser Führungsfläche kann die herkömmliche Knochenschraube 9 gegenüber der Lotrechten (entsprechend der Richtung der Gewindeachse) verkippt werden. Dies gibt eine Vielzahl von Möglichkeiten, die Knochenschraube 9 im Knochen festzulegen. Diese Verkippung ist nicht nur in einer Richtung entlang der Längsachse des Langloches hier möglich, sondern auch in Richtungen quer dazu, so dass sich letztlich ein Bereich von im wesentlichen 360° ergibt, in dem die Schraube gegenüber der Gewindeachse gekippt werden kann.

Eine solche herkömmliche Knochenschraube 9 kann mit der erfindungsgemäßen Knochenplatte 1 verwendet werden, um eine Kompressionswirkung zu erzielen. Nicht geeignet ist diese Knochenschraube allerdings, um eine winkelstabile Festlegung in Knochenplatte 1 und Knochen zu erzielen. Hierzu kann die erfindungsgemäß an die Knochenplatte angepasste Knochenschraube 11 verwendet werden, wie nachfolgend anhand der Figuren 5a) und 6 bis 8 beschrieben.

Der mit dem Gewinde 13 versehene Abschnitt des Schraubenkopfes 12 weist eine Außenkontur (eine Einhüllende der Gewindezüge) auf, die ebenfalls sphärisch und komplementär zu der Kontur der Führungsstruktur 8 ist. Auf diese Weise kann die Knochenschraube 11 in der Führungsstruktur 8 geführt werden, wie in vergleichbarer Weise die Knochenschraube 9 in der Führungsstruktur 8a. So kann auch die Knochenschraube 11 zunächst zum Erzielen einer Kompressionswirkung eingesetzt werden. Die Besonderheit der Knochenschraube 11 liegt jedoch darin, dass sie in dem Langloch 4 der Knochenplatte 1 lagestabil festgelegt werden kann. Diese Situation ist z.B. in Fig. 5a) dargestellt. In dieser Stellung greift das Gewinde 13 des Schraubenkopfes 12 in die Gewindezüge 5 in dem Langloch 4 ein, und die Anlagestruktur (Gegenstruktur) 14 liegt formschlüssig an der Haltestruktur 6 an. Durch das Zusammenwirken der Gewindezüge 5 mit dem Gewinde 13 wird die Anlagestruktur 14 fest gegen die Haltestruktur 6 gepresst, wobei anzumerken ist, dass die Haltestruktur 6 und die Gewindezüge 5 ebenso koaxial ausgebildet sind, wie das Gewinde 13 und die Anlagestruktur 14 an dem Schraubenkopf 12. Der Übergang des Schraubenkopfes 12, genauer des Gewindes 13 an dem Schraubenkopf 12 in die Gewindezüge 5 in dem Langloch 4 wird erleichtert und geführt durch den zunächst eintretenden Kontakt zwischen der Gegenstruktur 14 und der Haltestruktur 6, welcher Kontakt eine eindeutig geführte Bewegung vorgibt. So kann der Anfang des Gewindezuges des Gewindes 13 sicher in die Gewindezüge 5 überführt werden, so dass das Gewinde 13 letztlich in den Gewindezügen 5 eingreift, ohne zu verkanten.

In einer wie in Fig. 5a) gezeigten Position umgreifen die Gewindezüge 5 den Schraubenkopf 12 bzw. das Gewinde 13 an demselben in einer Ebene der Oberfläche 2 der Knochenplatte 1 gesehen vorzugsweise um maximal 180°. Gehalten wird die Schraube 11 in ihrer Position aufgrund eines durch die Verkippung der durch die Führungsstruktur 8 vorgegebenen Führungsbahn gegenüber der Gewindelängsachse erzielten Klemmeffekt und durch das Zusammenwirken der weiter umgreifenden Haltestruktur 6 mit der Gegenstruktur 14, wobei die Gegenstruktur 14 durch das Zusammenwirken von Gewindezügen 5 und Gewinde 13 axial in die Haltestruktur 6 gepresst wird.

Nur aufgrund dieser Tatsache ist es möglich, das Langloch 4 durchgehend und über die volle Länge alternativ für die Kompression oder für die winkelstabile Fixierung nutzbar zu gestalten.

### Bezugszeichenliste

- 1: Knochenplatte
- 2: Oberseite
- 3: Unterseite
- 4: Langloch
- 5: Gewindezüge
- 6: Haltestruktur
- 7: Einstich
- 8: Führungsstruktur
- 8a: Führungsstruktur
- 9: Knochenschraube
- 10: Schraubenkopf
- 11: Knochenschraube
- 12: Schraubenkopf
- 13: Gewinde
- 14: Gegenstruktur (Anlagestruktur)

- α: Winkel
- β: Winkel

## Patentansprüche

1. Knochenplatte mit einer für die Anlage am Knochen ausgebildeten Unterseite (3) und einer vom Knochen abgewandten Oberseite (2) sowie mit einer Mehrzahl von Löchern, durch die zur Verankerung Knochenschrauben (9, 11) mit dem Knochen in Eingriff bringbar sind, wobei wenigstens eines der Löcher als Langloch (4) ausgebildet ist, mit der längeren Achse in Richtung der Plattenlängsachse, wobei in einem Teilbereich des Seitenrandes des Längsloches Gewindezüge (5) vorgesehen sind, **dadurch gekennzeichnet, dass** die Gewindezüge (5) in einer Richtung quer zu der Ebene der Oberseite (2) gesehen nur über einen Teil der Tiefe des Langloches (4) angeordnet sind und sich in dieser Richtung gesehen ober- und/oder unterhalb der Gewindezüge (5) eine glattwandige Haltestruktur (6) zum formschlüssigen Eingriff mit einer entsprechend ausgestalteten Gegenstruktur (14) an einem Schraubenkopf (10, 12) bzw. -hals einer Knochenschraube (9, 11) befindet und dass die Gewindezüge (5) entlang eines kürzeren Umfangsabschnitt des Langloches verlaufen als die glattwandige Haltestruktur (6).

2. Knochenplatte nach Anspruch 1, **dadurch gekennzeichnet, dass** die Gewindezüge (5) in der Ebene der Oberfläche (3) gesehen einen Winkelbereich α eines kreisförmigen Umfanges eines mit diesem in Eingriff zu bringenden Schraubenkopfes (10, 12) bzw. -halses einer Knochenschraube (9, 11) umgreift, wobei gilt: 60° ≤ α ≤ 190°, vorzugsweise 60° ≤ α ≤ 180°.

3. Knochenplatte nach einem der vorhergehenden Ansprüche, **dadurch gekennzeichnet, dass** die glattwandige Haltestruktur (6) in der Ebene der Oberfläche (3) gesehen einen Winkelbereich β eines kreisförmigen Umfanges eines mit diesem in Eingriff zu bringenden Schraubenkopfes (10, 12) bzw. -halses einer Knochenschraube (9, 11) umgreift, wobei gilt: 185° ≤ β ≤ 300.

4. Knochenplatte nach einem der vorhergehenden Ansprüche, **dadurch gekennzeichnet, dass** die Haltestruktur (6) eine konische Fläche ist.

5. Knochenplatte nach einem der vorhergehenden Ansprüche, **dadurch gekennzeichnet, dass** die Gewindezüge (5) in einem nahe der Oberseite (2) der Knochenplatte (1) gelegenen Abschnitt angeordnet sind, die Haltestruktur (6) in einem nahe der Unterseite (3) angeordneten Abschnitt.

6. Knochenplatte nach einem der vorhergehenden Ansprüche, **dadurch gekennzeichnet, dass** das Langloch (4) auf der Oberseite (2) der Knochenplatte (1) eine erste Führungsstruktur (8) zur Führung des Kopfes (10, 12) oder Halses einer an ihrem Kopf und/oder Hals mit Gewindezügen und der Gegenstruktur versehenen Knochenschraube (9, 11) aufweist, wobei die erste Führungsstruktur (8) eine Führungsbahn definiert und diese Führungsbahn von einer Längsachse des durch die Gewindezüge (5) gebildeten Gewindes um einen von 90° verschiedenen Winkel geschnitten wird.

7. Knochenplatte nach Anspruch 6, **dadurch gekennzeichnet, dass** die Führungsbahn, um einen Winkel von größer 0° und kleiner 90° gegenüber der Plattenebene geneigt ist.

8. Knochenplatte nach Anspruch 7, **dadurch gekennzeichnet, dass** die Gewindezüge (5) an einer Schmalseite des Langloches (4), vorzugsweise symmetrisch zu dessen längerer Achse, angeordnet ist, wobei in diesem Bereich des Langloches (4) die erste Führungsstruktur (8) den größten Abstand zu der Oberseite (2) der Knochenplatte (1) aufweist.

9. Knochenplatte nach einem der Ansprüche 6 bis 8, **dadurch gekennzeichnet, dass** das Langloch (4) zusätzlich zu der ersten Führungsstruktur (8) eine zweite Führungsstruktur (8a) zur Führung von Knochenschrauben (9) mit gewindelosem Kopf (10) und Hals aufweist, wobei die zweite Führungsstruktur (8a) einen gegenüber der Plattenoberfläche geneigten Verlauf aufweist und der der Unterseite (3) der Knochenplatte (1) nächstgelegene Abschnitt der zweiten Führungsstruktur (8a) vorzugsweise in dem Bereich des Langloches (4) liegt, in dem die Gewindezüge (5) und die Haltestruktur (6) angeordnet sind.

## Claims

1. Bone plate with a bottom side (3) constructed for application to the bone and a top side (2) remote from the bone, as well as with a plurality of holes through which the bone screws (9, 11) can be engaged with the bone for anchoring purposes, at least one of the holes being constructed as an elongated hole (4), with the longer axis in the direction of the plate longitudinal axis and in a partial area of the lateral edge of the elongated hole thread grooves (5) are provided, **characterized in that**, considered in a direction at right angles to the plane of the top side (2), the thread grooves are only placed over part of the depth of the elongated hole (4) and, considered in this direction, above and/or below the thread grooves (5) there is a smooth walled holding structure (6) for positive engagement with a correspondingly designed counterstructure (14) on a screw head (10, 12) or neck of a bone screw (9, 11) and that the thread grooves (5) run along a shorter circumferential portion of the elongated hole than the smooth walled holding structure (6).

2. Bone plate according to claim 1, **characterized in that**, considered in the plane of the surface (3), the thread grooves embrace an angular range α of a circular circumference of a screw head (10, 12) or a screw neck of a bone screw (9, 11) to be engaged therewith, in which 60° ≤ α ≤ 190°, preferably 60° ≤ α ≤ 180°.

3. Bone plate according to one of the preceding claims, **characterized in that**, considered in the plane of the surface (3), the smooth walled holding structure (6) embraces an angular range of a circular circumference of a screw head (10, 12) or screw neck of a bone screw (9, 11) to be engaged therewith, in which 185° ≤ β ≤ 300°.

4. Bone plate according to one of the preceding claims, **characterized in that** the holding structure (6) is a conical surface.

5. Bone plate according to one of the preceding claims, **characterized in that** the thread grooves (5) are located in a portion close to the top side (2) of bone plate (1) and the holding structure (6) in a portion close to the bottom side (3).

6. Bone plate according to one of the preceding claims, **characterized in that**, on the top side (2) of bone plate (1), the elongated hole (4) has a first guide structure (8) for guiding head (10, 12) or neck of a bone screw (9, 11) provided on its head and/or neck with thread grooves and the counterstructure, the first guide structure (8) defining a guideway and said guideway is intersected by a longitudinal axis of the thread formed by the thread grooves (5) under an angle differing from 90°.

7. Bone plate according to claim 6, **characterized in that** the guideway is inclined relative to the plate plane by an angle greater than 0° and smaller than 90°.

8. Bone plate according to claim 7, **characterized in that** the thread grooves (5) are located on a narrow side of the elongated hole (4) and preferably symmetrical to its longer axis and in this area of the elongated hole (4) the first guide structure (8) has the maximum spacing from the top side (2) of bone plate (1).

9. Bone plate according to one of the claims 6 to 8, **characterized in that**, in addition to the first guide structure (8), the elongated hole (4) has a second guide structure (8a) for guiding the bone screw (9) with a threadless head (10) and neck, the second guide structure (8a) having an inclined path with respect to the plate surface and the portion of the second guide structure (8a) closest to the bottom side (3) of bone plate (1) is preferably located in the area of elongated hole (4) in which the thread grooves (5) and holding structure (6) are located.

## Revendications

1. Plaque d'ostéosynthèse comprenant un côté inférieur (3) formé pour l'appui sur l'os et un côté supérieur (2) opposé à l'os ainsi qu'une pluralité de trous, par lesquels des vis à os (9, 11) peuvent être mises en prise avec l'os pour l'ancrage, au moins l'un des trous étant conçu sous forme de trou oblong (4), avec l'axe plus long en direction de l'axe longitudinal de la plaque, des passages filetés (5) étant prévus dans une zone partielle du bord latéral du trou oblong, **caractérisée en ce que** les passages filetés (5), vus dans une direction transversale au plan du côté supérieur (2), sont disposés seulement sur une partie de la profondeur du trou oblong (4) et une structure de retenue (6) à paroi lisse pour l'engrènement par complémentarité de formes avec une contre-structure (14) de conception appropriée sur une tête de vis (10, 12) ou un collet de vis d'une vis à os (9, 11) se trouve, vue dans cette direction, au-dessus et/ou au-dessous des passages filetés (5), et **en ce que** les passages filetés (5) sont agencés le long d'une partie périphérique plus courte du trou oblong que de la structure de retenue (6) à paroi lisse.

2. Plaque d'ostéosynthèse selon la revendication 1, **caractérisée en ce que** les passages filetés (5), vus dans le plan de la surface (3), enveloppent une plage angulaire a d'un pourtour circulaire d'une tête de vis (10, 12) ou d'un collet de vis, à mettre en prise avec ce pourtour, d'une vis à os (9, 11), sachant que 60° ≤ α ≤ 190°, de préférence 60° ≤ α ≤ 180°.

3. Plaque d'ostéosynthèse selon l'une des revendications précédentes, **caractérisée en ce que** la structure de retenue (6) à paroi lisse, vue dans le plan de la surface (3), enveloppe une plage angulaire β d'un pourtour circulaire d'une tête de vis (10, 12) ou d'un collet de vis, à mettre en prise avec ce pourtour, d'une vis à os (9, 11), sachant que : 185° ≤ β ≤ 300°.

4. Plaque d'ostéosynthèse selon l'une des revendications précédentes, **caractérisée en ce que** la structure de retenue (6) est une surface conique.

5. Plaque d'ostéosynthèse selon l'une des revendications précédentes, **caractérisée en ce que** les passages filetés (5) sont disposés dans une partie située à proximité du côté supérieur (2) de la plaque d'ostéosynthèse (1), et la structure de retenue (6) dans une partie disposée à proximité du côté inférieur (3).

6. Plaque d'ostéosynthèse selon l'une des revendications précédentes, **caractérisée en ce que** le trou oblong (4) présente sur le côté supérieur (2) de la plaque d'ostéosynthèse (1) une première structure de guidage (8) pour le guidage de la tête (10, 12) ou du collet d'une vis à os (9, 11) dotée sur sa tête et/ou son collet de passages filetés et de la contre-structure, la première structure de guidage (8) définissant une bande de guidage et cette bande de guidage étant coupée par un axe longitudinal du filetage, formé par les passages filetés (5), sous un angle différent de 90°.

7. Plaque d'ostéosynthèse selon la revendication 6, **caractérisée en ce que** la bande de guidage est inclinée d'un angle supérieur à 0° et inférieur à 90° par rapport au plan de la plaque.

8. Plaque d'ostéosynthèse selon la revendication 7, **caractérisée en ce que** les passages filetés (5) sont disposés sur un côté étroit du trou oblong (4), de préférence symétrique par rapport à son axe plus long, la première structure de guidage (8) présentant dans cette zone du trou oblong (4) la plus grand distance au côté supérieur (2) de la plaque d'ostéosynthèse (1).

9. Plaque d'ostéosynthèse selon l'une des revendications 6 à 8, **caractérisée en ce que** le trou oblong (4) présente en supplément de la première structure de guidage (8) une seconde structure de guidage (8a) pour le guidage de vis à os (9) avec tête (10) et collet sans filetage, la seconde structure de guidage (8a) présentant un tracé incliné par rapport à la surface de la plaque et la partie, la plus proche du côté inférieur (3) de la plaque d'ostéosynthèse (1), de la seconde structure de guidage (8a) étant située de préférence dans la zone du trou oblong (4), dans laquelle sont disposés les passages filetés (5) et la structure de retenue (6).
